# EUROPEAN PATENT SPECIFICATION

(11) **EP 0 369 943 B1**
(45) Date of publication and mention of the grant of the patent: **28.09.1994**
(21) Application number: 89810845.1
(22) Date of filing: 08.11.1989
(51) Int. Cl.: C12N 15/12, C12N 15/18, C07K 13/00, C07H 21/04, C12N 5/10, C12P 21/00, A61K 37/02, A61K 37/36

(54) **Binding protein for insulin-like growth factors**
Bindeproteine für insulinähnliche Wachstumsfaktoren
Protéines de liaison pour facteurs de croissance semblables à l'insuline

(30) Priority: 11.11.1988 GB 8826451
(43) Date of publication of application: 23.05.1990
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Binkert, Christopher, CH-4055 Basel (CH); Heinrich, Günther, F., CH-4125 Riehen (CH); Schwander, Jürg, CH-4059 Basel (CH)

(56) References cited:
- WO-A-89/08667
- WO-A-89/09792
- THE EMBO JOURNAL, vol. 7, no. 8, August 1988, Oxford, GB A. BRINKMANN et al."Isolation and character- ization of a cDNA encoding the low modecular weightinsulin-like growth factor binding protein (IBP-1)" pages 2417-2423
- BIOCHEMICAL AND BIOPHYSICAL RESEARCH COMMUNICATIONS, vol. 152, no. 3, May 16,1988, San Diego, USA M.T. BREWER et al. "Cloning, Characterization andExpression of a Human Insuline-line Growth Factor Binding Protein" pages 1289-1297
- FEBS, EUROPEAN JOURNAL OF BIOCHEMISTRY, vol. 144, no. 1, October 1984, BerlinG. POVOA et al. "Isolation and characterization of a somatomedin-bindingprotein from mid-term human amniotic fluid" pages 199-204
- ENDOCRINOLOGY, vol. 118, no. 4, April 1986, Springfield, USA R. KOISTINEN etal. "Placental Protein 12 Is a Decidual Protein that Binds Somatomedin and Hasan Identical N-Terminal Amino Acid Sequence with Somatomedin-Binding Proteinfrom Human Amniotic Fluid" pages 1375-1378
- JOURNAL OF CHROMATOGRAPHY, vol. 420, 1987, Amsterdam D.R. POWELL "Method forpurification of an insulin -like growth factor-binding protein produced byhuman HEB G2 hepatoma cells" pages 163-170
- BIOCHEMICAL AND BIOPHYSICAL RESEARCH COMMUNICATIONS, vol. 139, no. 3, September30, 1986, San Diego, USA R.C. BAXTER et al. "Growth Hormone-Dependent Insulin -like Growth Factor (IGF) Binding Protein from Human Plasma Differs from Other
- Human IGF Binding Proteins" pages 1256-1261
- THE JOURNAL OF BIOLOGICAL CHEMISTRY, vol. 261, no. 24, August 25, 1986,Baltimore, USA C. MOTTOLA et al. "Purification and Amino -terminal Sequence ofan Insulin-like Growth Factor -binding Protein Secreted by Rat Liver BRL-3ACells" pages 11 180-11 188

## Description

This invention relates to carrier proteins that bind to Insulin-like Growth Factors (IGF's), also known as somatomedins, a process for producing them and their use in various therapeutic applications.

IGFs are low molecular weight polypeptide hormones with structural homology to proinsulin. Two different IGFs are known, namely IGF-I and IGF-II which are mitogenic in vitro for a wide variety of cells in tissue culture. Both IGFs stimulate in vivo the growth of various tissues and in particular they induce collagen synthesis. IGF-I mediates the growth promoting effect of growth hormone in chondrogenesis and bone formation and is therefore essential for normal growth of an individual. This is demonstrated by the fact that pygmies and toy poodles are deficient in IGF-I but have normal growth hormone level in their serum. IGF-II is believed to play a key role in fetal development and nerve growth.

In addition to their primary effect on skeletal tissue they also assume growth-stimulating functions on other tissues. Wound fibroblasts are known to produce IGFs which are effective in stimulating fibroblasts to grow and synthetize collagen, a structural protein normally required for wound healing. Vascularisation of the wound tissue is also induced. Further, it has also been found that IGFs have an erythropoetin-like activity in that they induce hematopoesis.

Recent studies have also demonstrated that IGFs produced by certain cancer cells, e.g. breast and kidney cancer cells auto-stimulate the proliferation of cancer cells and the vascular and fibrous tissues required to support the growth of cancer tissues.

In addition to this, both IGFs show a spectrum of metabolic activities similar to those of insulin, in that they stimulate, in particular, the transport and metabolism of glucose. The biological effects of IGFs and insulin are mediated through their binding to specific receptors. In particular, both IGFs have the ability to bind to the insulin receptor with approximately 100 fold lower affinity than does insulin.

Both IGFs have a concentration in blood approximately a hundred-fold higher than that of insulin. Because IGF's have insulin-like activity, this should lead therefore to hypoglycemia. This is prevented by a regulatory mechanism which involves carrier proteins present in blood and able to form complexes with IGFs. Thus, IGFs circulate in the blood in the form of a complex which has no insulin-like activity. Through their association with carrier protein (hereinafter referred to as IGF-binding proteins or IGF-BPs), binding to their cell surface receptors is inhibited. It has also been demonstrated that another function of the IGF-binding proteins is to increase the short half-life of IGFs which are subjected to rapid proteolytic degradation when present in the free form in blood.

In accordance with the foregoing, IGFs may be useful in vivo to stimulate a) the growth of animals and humans with growth hormone deficiency, b) tissue regeneration, such as erythropoiesis and chondrogenesis, c) wound healing and d) the functions of various organs e.g. liver or kidney. As a result of their chondrogenesis stimulating activity, IGFs are of particularly suitable use for bone formation, e.g. in the treatment of osteoporosis. IGF's for use in the above-referred treatments are advantageously administered to a subject in association with at least one IGF-binding protein. Administration of this combination rather than IGF alone has beneficial effects including the prevention of hypoglycemia and possible mitogenic effects at injection sites and the prolongation of IGF half-life. Further, it has now been found that binding proteins are also useful for potentiating the erythropoetin like-effect of IGF-I.

When administered alone, i.e. without any IGF, the binding proteins may also be therapeutically useful for blocking the adverse effects of IGFs, such as those which occur when IGFs are produced in excess, e.g. free IGFs secreted by certain cancer cells e.g. hormone-producing cancer cells such as breast or kidney cancer cells. IGF-binding therapy may also prevent blindness as a secondary effect of diabetic proliferation retinopathy. Indeed it has been shown that IGFs may be one of the factors stimulating endothelial and fibroblast proliferation in diabetic retinopathy.

Another therapeutic use is the control of excessive growth in IGF-binding protein deficient subjects since it is very likely that high IGF levels combined with abnormally low levels of binding protein are responsible for excessive growth.

In recent years, at least two major species of IGF-binding proteins, different in size, have been detected in the serum of rodents and humans.

The large binding protein is a glycoprotein of approximately 150 kd and composed of several subunits. Its formation in contrast to that of the small binding protein is growth hormone dependent.

The small binding protein has a molecular weight of approximately 30 - 40 kd in the human and the rat. The human small binding protein has already been purified from various sources including amniotic fluid (Povoa, G. et al Eur. J. Biochem (1984) 144 : 199, (therefore also referred to as amniotic fluid binding protein), placenta (Koistenen, R. et al Endocrinology (1986) 118 : 1375) and conditioned medium of hepatoma G2 cells (Powell, D.R. et al J. Chromatogr. (1987) 420 : 163). The binding proteins have been characterized by their amino acid contents and their N-terminal amino acid sequences, and found identical or at least very similar. The comparison of the amino acid sequences of the IGF-binding protein isolated from hepatoma G2 cells (Lee, Y.L. et al Mol. Endocrinol. (1988) 2 (5) : 404) and the IGF-binding protein cloned from a placenta cDNA library (Brinkman, A. et al. The EMBO Journal (1988) 7 (8) : 2417) reveals 99 % homology. Further, these two amino acid sequences show with the IGF-binding protein as coded by a cDNA sequence cloned from a human uterus cDNA library a homology of 94 % (Brewer, M.T. et al Bioch. Biophys. Res. Com. (1988) 152 (3) : 1289).

In addition to the two major forms of IGF-binding proteins present in serum several other IGF-binding proteins have been identified in different human tissue extracts and cell culture media by Western blotting techniques and affinity labelling with [I¹²⁵] IGF. Their molecular weights range from 15 to 150 kd and some of these proteins may be generated by proteolytic degradation of the larger IGF-binding-proteins. In particular, a 53 kd IGF-binding protein which has been purified from human serum represents a subunit of the 150 kd IGF-binding protein (Baxter, R.C. Biochem. Biophys. Res. Com. (1986) 139 (3) : 1256).

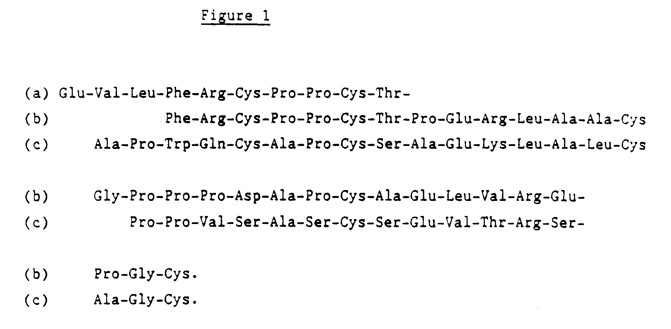

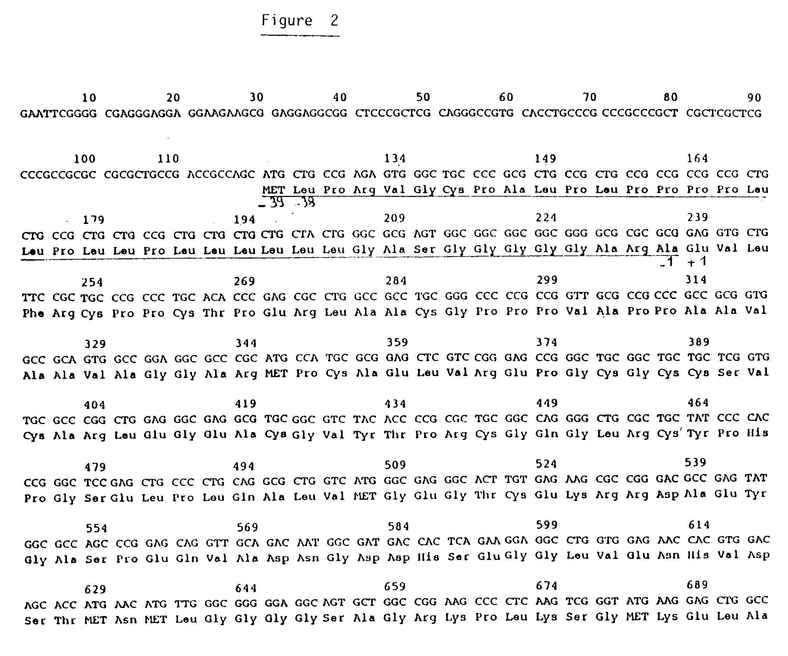

Another form of IGF-binding protein has also been found in the conditioned medium from rat BRL-3A cells, and has a molecular weight of 33-36 kd approximately. A partial amino-terminal protein sequence of the rat BRL-3A binding protein has been determined (Mottola, C. et al J. of Biol. Chem. (1986) 261 : 11180; Lyons, R.M., Smith, G.L. Mol. Cell. Endocrinol. (1986) 45 : 263) and is shown in Figure 1 together with the N-terminal amino acid sequence of the human small binding protein (Povoa, G. et al and Lee. X.L. et al). The 33 % degree of homology shown by the rat and human terminal sequences is not high enough to allow the respective binding proteins to be considered as equivalents.

The human binding-protein equivalent to that of the rat BRL-3A cells has now been found and its amino acid sequence fully established. Comparison with the sequence of other small human binding proteins clearly indicates that this new human binding protein represents a distinctive form.

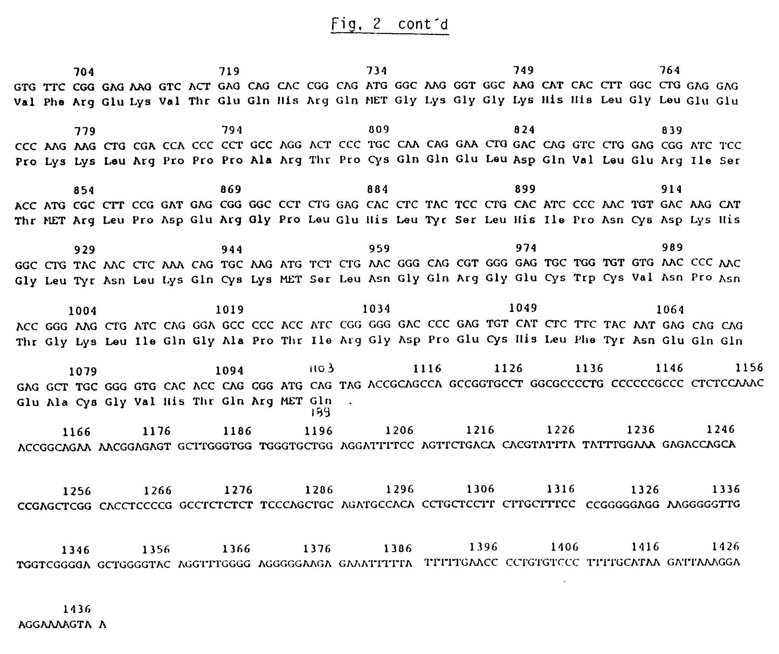

Accordingly, the present invention provides a new IGF-binding protein comprising the 289 amino acid sequence shown in Figure 2 beginning with glutamic acid at position 1 (N-terminal residue) and ending with glutamine at position 289 (C-terminal residue), also called mature IGF-BP. Preferably, this protein consists of this 289 amino acid sequence. The protein may also comprise the 327 or 328 amino acid sequence beginning with leucine at position -38 or with methionine at position -39 and ending with glutamine at position 289, which sequence corresponds to the precursor form of the 289 amino acid mature protein as it contains the signal sequence together with the complete sequence of the mature protein. Within the scope of the invention is also included any allelic form of the precursor or mature protein which amino acid sequence departs from that shown in Figure 2 by deletion, addition or substitution of at least one amino acid, so long as at least one of the functional properties is maintained. For example, such variations may increase or decrease the IGF-binding affinity of the protein. Deletions are characterized by the elimination of amino acid residues without the insertion of a replacement residue. Substitution mutants are those in which one amino acid residue has been replaced by another, whereas insertional mutants are those in which one or more residues are placed into or at either end of the amino acid sequence of the original protein. Fusions typically are a species of insertional mutant in which the insertion occurs at the carboxyl or amino terminal residue.

Since comparison between peptides may be conveniently established based on the degree of homology of their amino acid sequences, it is considered that the scope of the invention encompasses any protein which is substantially homologous i.e. being from 95 % to 100 % homologous with pre-IGF-BP or IGF-BP, preferably IGF-BP, as shown in Figure 2.

The ability of binding to an insulin-like growth factor is one of the biological activities of the proteins of the invention. These proteins may be conveniently tested in a binding assay using IGF-I [Rinderknecht, E. and Humbel, R.E., J. Biol. Chem. (1978) 253: 2769] or IGF-II [Rinderknecht, E. and Humbel, R.E., FEBS (1978) 89: 283], preferably IGF-II, in a labelled, e.g. iodinated form. For example, such an assay may conveniently include performing a gel electrophoresis (SDS-PAGE) of the proteins of the invention, followed by a Western blot of the gel, then incubating the blot in the presence of [¹²⁵I]IGF-I or -II, washing the blot to remove free IGF-I or -II, and detecting the radioactivity on the blot.

While IGF-BPs of the invention originally means human IGF-BPs, IGF-BPs of the invention from sources such as mammalian, e.g. murine, porcine, equine or bovine are included within the definition of IGF-BPs as long as they comply with the required degree of homology.

The IGF-BPs of the invention include those purified from a tissue extract or from a conditioned culture medium as well as those obtained by recombinant means.

Derivatives of IGF-BPs are included within the scope of this invention. Derivatives include glycosylated forms, aggregative conjugates with other IGF-BP molecules and covalent conjugates with unrelated chemical moieties. Covalent derivatives are prepared by linkage of functionalities to groups which are found in the IGF-BP amino acid chain or at the N- or C-terminal residue by means known in the art.

The invention further provides recombinant nucleic acid sequences encoding the IGF-BPs of the invention, e.g. the protein comprising the 289 amino acid sequence shown in Figure 2 beginning with glutamic acid at position 1 and ending with glutamine at position 289. The nucleic acid sequence may encode the precursor protein shown in Figure 2 beginning with leucine at position -38 or with methionine at position -39 and ending with glutamine at position 289. Within the scope of the invention it is also included any nucleic acid sequence encoding allelic forms of the precursor or mature IGF-BP as shown in Figure 2. By the term recombinant nucleic acid sequence is meant any nucleic acid sequence produced by joining pieces of nucleic acid sequences from different sources. By extension this term also includes a sequence isolated from its native origin. These recombinant nucleic acid sequences may be mRNA or rDNA including cDNA and genomic rDNA. Preferably they are cDNA. Most preferably the cDNA sequence is that of Figure 2 starting either at position 120, 123 or 237 and ending at position 1103.

As a general guide, any nucleic acid sequence which hybridizes with the DNA sequence encoding pre- or mature IGF-BP of Figure 2 under stringent hybridization condition, e.g. at 65°C in 4X saline citrate buffer for no longer than 16 hrs is considered to fall under the scope of the invention.

The general strategy whereby the illustrated IGF-BP encoding sequences were obtained is as follows:

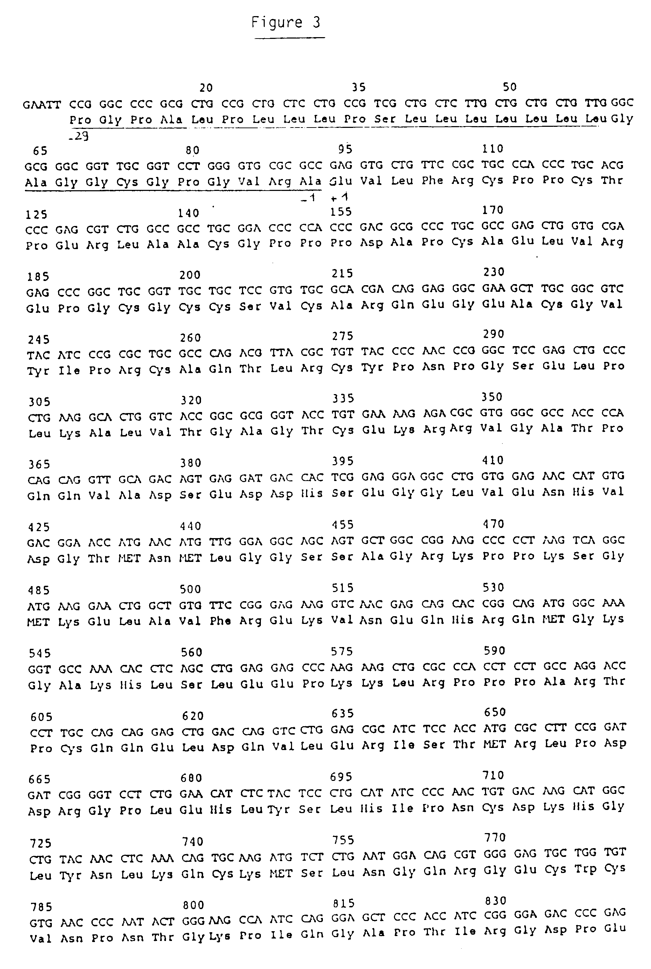

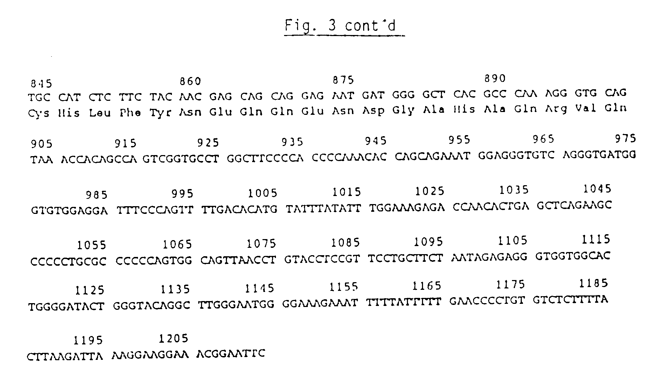

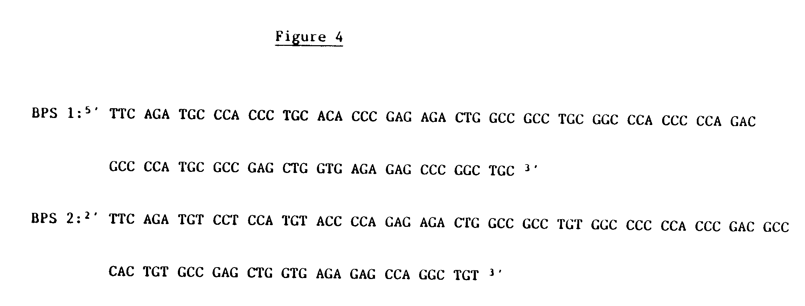

The known N-terminal sequence of the rat BRL-3A cell IGF-BP as shown in Figure 1 was used to design two 92-base oligonuclectide probes called BPS 1 and BPS 2 which are shown in Figure 4. After radiolabelling, these probes were used to screen a rat cDNA library. 34 independent cross-hybridizable clones out of 10⁶ clones were detected and analysed by restriction enzyme and Southern blot analysis. These 34 clones were classified into 12 different clone groups, according to the cDNA fragment size. The cDNA fragments representative of each group were cloned in the vector M13mp8 (Vieira, J. and Messing, J. Gene (1982) 19 : 259) and sequenced. As a result, the complete nucleotide sequence of the rat BRL-3A IGF-BP was determined from one of the groups as shown in Figure 3. It has an open reading frame encoding 299 amino acids. If the NH₂-terminus of the mature binding protein corresponds to the sequence determined by Lyons, R.M. and Smith, G.L., then the binding protein and the signal peptide are composed of 270 and 29 amino acids respectively. The protein has no N-glycosylation sites and contains 19 cystein residues most of these being clustered close to its NH₂ terminus.

Three human cDNA libraries were screened with the rat cDNA sequence as probe : an adult liver library (commercially available at Clontech under the catalogue number HL 10013) a fetal liver library (Clontech, HL 1005) and a cDNA library derived from the HEP G2 cell line (Clontech, HL 1015). Although 5x10⁵ independent phage clones from the adult liver cDNA library were screened, no hybridizable clone could be detected . Out of 2,5 x 10⁵ (fetal human liver library) and 1,5x10⁵ (HEP G2 library) clones, 14 and 3 cross hybridizable clones were respectively isolated and analysed. The cDNA inserts of the 3 HEP G2 clones were found identical and approximately 800 bp long. This nucleotide sequence was also encoded on a 1,4 kb long cDNA clone isolated from the fetal liver cDNA library, which sequence was further determined, as disclosed in Figure 2. This cDNA has an open reading frame encoding 328 amino acids starting with the translational codon ATG. Amino acid comparison with the rat BRL 3A binding protein reveals a homology of 81 %.

The mature human binding-protein encoded by this DNA sequence is 19 amino acids longer than the rat counterpart if the signal peptide is cleaved at the same position as in the rat BRL-3A binding-protein. This human binding protein and the other 30 kd human binding protein (Lee, Y. L. et al; Brinkman, A. et al and Brewer, M.T. et al) have approximately 37 % of their amino acids in common. This low degree of homology clearly reveals that they are different binding proteins. Two structures in the sequence are shared with the already known binding proteins, i.e. a cysteine rich region at the NH₂ terminus of the protein and an Arg-Gly-Asp sequence at positions 262-264. The latter sequence distinguishes this binding protein from other human hormone-binding proteins and may be important for its function, e.g. binding to its specific cell surface receptor.

Another aspect of the invention provides expression vectors comprising a DNA sequence encoding an IGF-BP of the invention, which is operatively linked to appropriate control sequences allowing the expression of said IGF-BP in a suitable eucaryotic or procaryotic host. Such control sequences include a transcriptional promoter, an optional operator to control transcription, a sequence encoding suitable mRNA ribosomal binding sites and sequences which control termination of transcription and translation. Vectors include plasmids, viruses and integratable DNA fragments, i.e. fragments that are integratable in the host genome by recombination. For use in the present invention, plasmids are advantageously chosen. They may be suitable for expression either in a procaryotic or eucaryotic host, the latter being preferred.

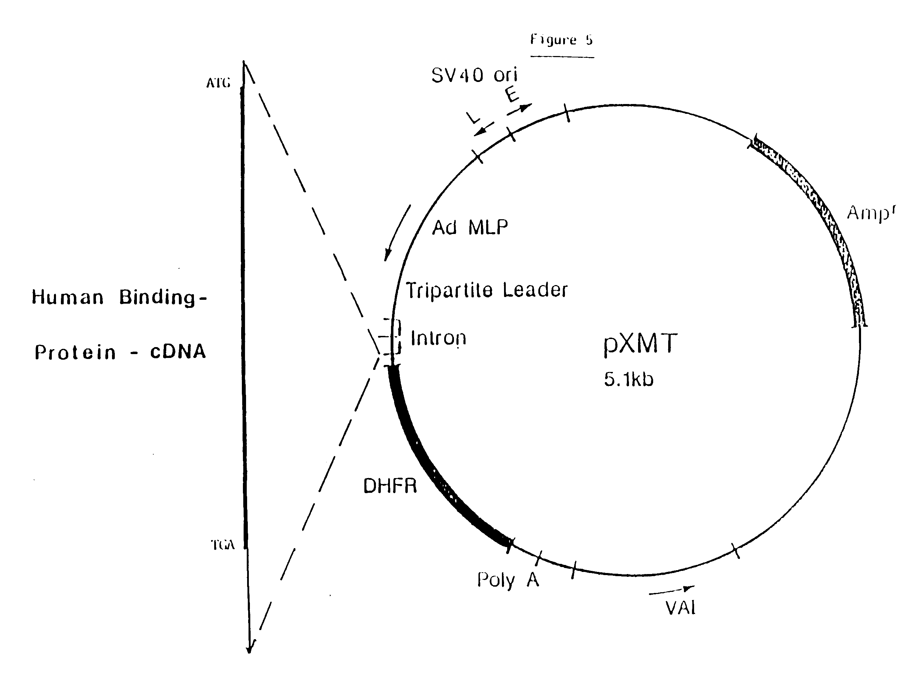

In a preferred embodiment of the invention, an EcoRI - EcoRI cDNA fragment encoding the precursor binding protein as shown in Figure 2 is cloned in plasmid pXMT previously digested by EcoRI. Plasmid pXMT is shown in Figure 5 and was derived from p91023(B) (Wong, G.G. et al, Science 228 : 810) by standard DNA manipulations. pXMT has the pBR322 origin of replication and ampicilline resistance gene for propagation in E.coli and lacks the bacterial sequences which inhibit DNA replication in COS monkey cells. It contains, in addition, eukaryotic regulatory elements from several different sources; (i) an SV40 origin and SV40 enhancer segment which together allow the plasmid to replicate to a very high copy number in COS cells and to efficiently transcribe inserted cDNA genes; (ii) the adenovirus major late promoter coupled to a cDNA copy of the adenovirus tripartite leader; (iii) a hybrid intron consisting of a 5′ splice site from the first exon of the tripartite leader and a 3′ splice site from a mouse immunoglobulin gene; (iv) the SV40 early polyadenylation signal; and (v) the adenovirus VA I gene region. Plasmid pXMT also contains the mouse dihydrofolate reductase (DHFR) coding sequence downstream from the unique Eco RI cloning site which is positioned just after the 3′ splice site.

In accordance with the present invention, cell lines are also provided which have been transformed with a DNA sequence or a vector according to the invention. The selection of suitable host cells as well as methods for transformation and growth are known in the art. Whereas procaryotic and eucaryotic cell-lines may be of use, the latter are preferred. Eucaryotic cells comprise, for example, yeast cells or cells derived from a multicellular organism e.g. mammalian cells. One particularly suitable mammalian cell line is the Chinese Hamster ovary (CHO) cell-line. Another suitable mammalian cell-line is the monkey COS-1 or CV-1 cell-line.

According to another aspect of the present invention there is provided a method for the production of IGF-BP which comprises recovering said IGF-BP from a culture of a cell line transformed witha DNA fragment or a vector of the invention.

### Brief description of the drawings

Figure 1 is a comparison of the N-terminal sequence of the rat BRL-3A binding-protein as determined by (a) Lyons, R.M. and Smith, G.L. Mol. Cell. Endocrinol. (1986) 45 : 263; or (b) Mottola, C. et al J. of Biol. Chem. (1986) 261 : 11180, and the N-terminal sequence of the human small binding protein (c) as determined by Lee, Y.L. et al Mol. Endocrinol. (1988) 2 (5) 404 ; Brinkman, A. et al The EMBO Journal (1988) 7 (8) : 2417; or Povoa, G. et al Eur. J. Biochem. (1984) 144 : 119. A gap is introduced to maximize the sequence homology between the two proteins.

Figure 2 shows the sequence of an EcoRI-EcoRI cDNA fragment cloned from a fetal liver cDNA library (Clontech, HL 1005) and the corresponding amino acid sequence of the precursor form of the human binding protein equivalent to that of the rat BRL-3A cells beginning at position -39 and ending at position 289. The mature protein begins at position 1. The signal sequence is underlined.

Figure 3 shows the complete cDNA sequence of the rat BRL-3A binding-protein encoding the precursor form of the protein beginning at position -29 and the mature form beginning at position 1. The signal sequence is underlined.

Figure 4 states the DNA sequences of the probes BPS-1 and BPS-2 designed according to the N-terminal sequence of the rat BRL-3A binding protein as reported by Mottola et al. and used for screening rat cDNA libraries.

Figure 5 depicts plasmid pXMT and its derivatives obtained after insertion of a cDNA fragment into the unique EcoRI site.

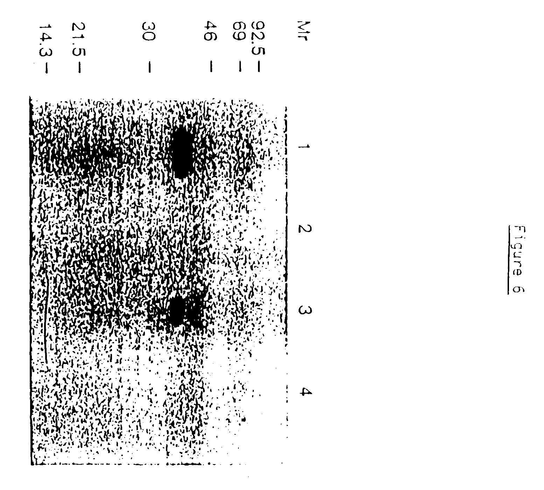

Figure 6 shows a Western blot analysis obtained as follows: 50 »l of medium conditioned by untransfected (lane 4), pXMT+ (lane 1) or pXMT- (lane 2) transfected CHO cells were applied to polycrylamide gel electrophoresis under non-reducing conditions. On lane 3, 50 »l of human umbilical cord serum was applied. After migration, proteins were then transferred to nitrocellulose membranes and exposed to [¹²⁵I]-IGF-I.

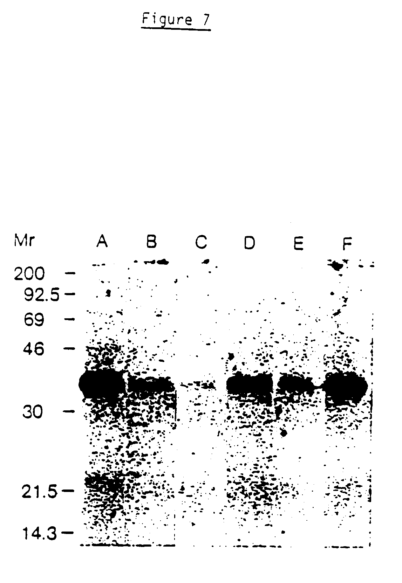

Figure 7 shows a Western block analysis of medium conditioned by pXMT+ transfected CHO cells. The binding of 1 ng [¹²⁵I]-IGF-II was competed with 0 ng (lane A), 5 ng (lane B), 25 ng (lane C) of unlabelled IGF-II; 5 ng or 25 ng (lane D and E) of cold IGF-I or 100 ng (lane F) of unlabelled insulin. The blot was prepared as in Figure 6.

### Example 1

### Recovery of a cDNA encoding rat IGF binding protein.

Two 92-base oligonucleotide probes, BPS-1 and BPS-2, which correspond to the N-terminal sequence of the BRL-3A binding protein determined by Mottola et al were synthetized on an Applied Biosystems 380 A oligonucleotide synthesizer. These probes are shown in Figure 4. They were designed by choosing the codons for the corresponding amino acids which occur most frequently in the rat. The probes were then labelled with γ[³²P]ATP using polynucleotide kinase.

10⁶ recombinant λgt11 phage clones from a post natal rat liver cDNA library (Skoder Biozentrum) or an adult rat liver cDNA library (Clontech, RL 1001) were screened using both probes as follows:

5 x 10⁴ recombinant λgt11 phages from each library were used to transfect 4.10⁹ E.coli K803 competent cells in 2 ml 10mM CaCl₂ · 10mM MgCl₂. After 15 mn incubation in a 37°C water bath, 30 ml of Luria Broth (LB) medium 1% soft agar was added to the cells and plated on 23 x 23 cm Nunclon petri dishes containing 400 ml of LB medium 1.5% agar. Plates were incubated for 12 hrs at 37°C and then transferred at 4°C for at least 1 hr.

A 21.5 x 22 cm Nitrocellulose membrane filter (Schleier and Schuell BA 85) was layed on the clones of each Petri dish and then transferred into 0.5M NaOH, 1.5M NaCl for 5 min to denature the DNA on the filters and washed twice in a neutralizing solution (0.2M Tris, 2.5M NaCl, 0.015M sodium citrate, 0.015M KH₂PO₄ and 0.001M EDTA(Na)₂, pH7). Finally the filters were air-dried for 1 hr before they were transferred in a vacuum oven for 2 hrs at 80°C.

Filters were then presoaked at 50°C for 6 hrs in 6xSSC, 1xDenhardt, 50 mM Pipes and 0.1M KPO₄ buffer, pH7. (20xSSC: 0.15M NaCl and 0.015M sodium citrate; 20xDenhardt: 0.4% bovine serum albumine, 0.4% ficoll and 0.4% Polyvinylpyrrolidone MW 360 Kd).

Hybridization was performed at 50°C for 36 hrs in 6xSSC, 1xDenhardt, 1mM Tris-HCl pH7, 0.2mM EDTA pH8, 0.9M KPO₄ pH7, 0.5% SDS complemented with 10 mg/ml heat-denatured salmon sperm DNA using a mixture of 10⁶ cpm/ml of probe BPS-1 and 10⁶ cpm/ml of BPS-2. Finally the filters were washed three times for 30 min each with 4xSSC, 0.5% SDS at 50°C and exposed with a Kodak X-OMAT XAR film.

The areas corresponding to the radioactive spots on the film were identified on the plates and picked out. After rescreening, 34 independent cross hybridizable clones were isolated from the liver cDNA libraries. These clones were analysed by restriction enzymes and Southern blot and classified into 12 groups. The cDNA insert representative of each group was cloned into M13mp8 (Vieira, J. and Messing, J. Gene (1982) 19 : 259) and sequenced according to the method of Sanger. The nucleotide sequence of 4 clones isolated from the post natal liver cDNA library and also sequenced were identical and corresponded to the partial protein sequence of the rat BRL-3A binding protein. The complete DNA sequence of Figure 3 was determined by joining sequences of overlapping cDNA subfragments. These subfragments were cloned and sequenced after random disruption of the original cDNA by sonication.

### Example 2

### Recovery of cDNA encoding human IGF-binding protein.

The complete cDNA fragment of Figure 3 encoding the rat BRL-3A binding protein was labelled using the random primer procedure (Boehringer Kit) and used for screening an adult human liver (Clontech, HL 10013) a fetal human liver (Clontech, HL 1005) and a HEP G2 cell (Clontech HL 1015) cDNA library. The screening procedure was identical to that described in Example 1 except that hybridization was performed at 65°C. Although 5 x 10⁵ independent phage clones from the adult liver cDNA library were screened, no cross-hybridizable phage clone could be detected. Out of 2.5 x 10⁵ (fetal human liver library) and 1.5 x 10⁵ phage clones (HEP G2) 14 and 3 cross-hybridizable clones respectively were isolated. The insert length of all HEP G2 specific cDNA clones was approx. 800 bp. The nucleotide sequence of these three cDNA clones was identical and was also encoded by a 1.3 kb long cDNA clone isolated from the fetal liver cDNA library. This 1.3 kb cDNA fragment was sequenced according to the method of Sanger using oligonucleotide primers and is to be seen in Figure 2.

### Example 3

### Expression of human IGF-binding protein.

An EcoRI-EcoRI fragment containing the cDNA sequence encoding the precursor binding protein as shown in Figure 2 was cloned into pXMT previously digested with EcoRI. After ligation a recombinant plasmid with an EcoRI-EcoRI fragment in proper orientation was identified and further digested with SsPI to linearize the vector.

10»g of the plasmid preparation so obtained was co-transfected with 1 »g circular plasmid pSV2-neo (Southern, P.J. and Berg, P. J. Mol. Applied Gen. (1982) 1 : 327) into 10⁶ CHO cells previously grown to 70% confluency in alpha⁺ MEM medium (available at Gibco under catalogue number 041-02571 M) and complemented with 10% fetal calf serum (FCS). The transfection was performed according to the scrape loading method of Fechheimer, M. et al PNAS (1987) 84 : 8463. Following the transfection the cells were seeded into 75 cm² culture flasks in alpha⁺ MEM medium complemented with 10% FCS.

After 48 hours, 1 mg/ml of the drug G418 was added to the medium. 18 days later the growing (drug resistant) colonies were transferred into alpha⁻ MEM medium (Gibco, 041-02561 H) complemented with 10% dialysed FCS.

The drug-resistant colonies as well as untransfected CHO cells used as control were then tested for their expression of the IGF-BP as follows: mRNA preparation were obtained and Northern blot analysis was performed using the DNA sequence encoding the NH₂ terminal sequence of the rat BRL-3A binding protein as probe. Hybridization was observed. CHO cells transfected with pXMT containing the cDNA insert in correct orientation (pXMT+) and 20 days old rat embryo cells transcribe a binding protein specific cDNA whereas CHO cells untransfected or transfected with pXMT containing the cDNA insert in wrong orientation (pXMT-) do not.

The supernatants of cultures of transfected and untransfected CHO (pXMT-) cells were further concentrated 20 fold and then separated on SDS-PAGE. Then Western blot analysis was performed. This blot was finally hybridized with [¹²⁵I]IGF-I as shown in Figure 6. In medium conditioned with CHO cells transfected with pXMT+ (lane 1) and in human serum (lane 3) a band of MW 36 Kd emerged. No signal was detected in media conditioned by untransfected CHO cells (lane 4) or by cells transfected with pXMT- (lane 2). This additionally demonstrates that only CHO cells transfected with the cDNA in the proper orientation expressed the binding protein.

To determine the ability of IBP-2 expressed by CHO cells to bind to IGF-II and its relative affinities for IGF-I, IGF-II and insulin, competitive binding studies were performed. 50 »l samples of conditioned medium from CHO cells transfected with pXMT+ were subjected to SDS-PAGE under non-reducing conditions and transferred onto nitrocellulose. The filter was cut vertically into strips and each strip was treated differently in a competitive binding assay (Figure 7). Each filter was hybridized to 1 ng [¹²⁵I]IGF-II (10⁶ cpm/5»g protein). Then, in lane A no unlabelled IGF-II, in lane B 5ng and in lane C 25ng unlabelled IGF-II were added additionally. When using IGF-I as competitor, 5ng (lane D) and 25ng (lane E) were added to the hybridization solution. 100 ng insulin were used in competition experiment F. The results of these competition experiments are illustrated in Figure 7. They show that the IGF-BP of the invention specifically binds IGF-II because the binding can be inhibited by only 5-fold excess of unlabelled IGF-II. The binding can also be inhibited by excess of unlabelled IGF-I, but IGF-I has a lower affinity than IGF-II. With a 100-fold excess of cold insulin no inhibition of [¹²⁵I]IGF-II binding was detected. This demonstrates that the human IGF-BP of the invention has a higher affinity for IGF-II than for IGF-I and does not bind to insulin. Also in this respect, the IGF-BP of the invention is different from the IGF-BP purified from human amniotic fluid which has higher or similar affinity for IGF-I.

### Example 4: Purification of the human IGF-binding protein

### Procedure 4A)

A CHO clone expressing the binding protein was seeded at a cell density of 2 to 4 x 10⁶ cells / 75 cm² flask and grown to 90 % confluency in alpha⁻ MEM medium. The cells were separated from the growth medium by filtration and the cell-free supernatant was used for the purification of IGF-binding protein.

200 ml of supernatant were diluted with an equal volume of 50 mM sodium phosphate buffer, pH 6.0 and the pH of the final solution was adjusted to pH 6.0 with phosphoric acid. The solution was then applied to a Q-Sepharose FF (Pharmacia) ion exchange column (10 x 120 mm) previously equilibrated with 50 mM sodium phosphate buffer, pH 6.0. The column was operated at a flow rate of 1.3 ml/min. The flow through of the column contained IGF-binding protein and was collected. Detection of the binding activity was by Western blotting i.e. fractions were subjected to SDS-PAGE electrophoresis, transferred to nitrocellulose sheets and the binding protein developed by its property to interact with radioactively labelled IGF.

To the flow through of the Q-Sepharose column containing the IGF-binding activity, trifluoroacetic acid (TFA) was added to give a final concentration of 0.1%. This acidified solution was subjected to further purification by reverse phase chromatography on a Vydac C4 column (4.6 x 250 mm) equilibrated with 0.1% TFA in water. Following application, proteins were eluted by gradient elution with a gradient of 0.1% TFA in water to 0.1% TFA in acetonitrole. Fractions containing the IGF-binding protein were eluted at about 30% acetonitrile as detected by Western blotting.

### Procedure 4B)

A cell culture supernatant containing IGF-BP is diluted 1:1 with 20 mM Tris-sodium-acetate, pH 5.5. After dilution, the pH of the solution is controlled and if necessary, adjusted to pH 5.5 with hydrochloric acid.

This solution is applied to a S-Sepharose (Pharmacia) column equilibrated with 20 mM Tris-sodium-acetate, pH 5.5. The column is washed with 20 mM Tris-sodium-acetate, pH 5.5 until the adsorbance has returned to base-line. IGF-BP is eluted by step-wise addition of sodium chloride in 100 mM increments to the 20 mM Tris-sodium-acetate, pH 5.5. IGF-BP elutes between 0.1 and 0.3 M sodium chloride, as detected by Western Blotting of the eluted fractions.

The fractions with IGF-binding activity are pooled and concentrated on an Amicon Concentrator, equipped with YM10 membranes. The concentrate is submitted to gelfiltration on a Superose 12 (Pharmacia) column equilibrated in 20 mM Tris-sodium-acetate, pH 5.5 buffer. Active fractions are pooled.

The pooled fractons containing IGF-BP from gelfiltration are subjected to reversed-phase chromatography on a Vydac C4 column previously equilibrated with 0.1% trifluoroacetic acid. The pooled Superose 12 eluate is applied and the column is washed to base-line with trifluoroacetic acid. IGF-BP is subsequently recovered from the column by gradient elution with acetonitrile containing 0.1% trifluoroacetic acid.

Therapeutic applications of the binding proteins of the invention include its use as a single therapeutic agent and its use in combination with an IGF, the latter use being preferred.

When used in combination with an IGF, a binding protein of the invention is suitable for use in the indications above mentioned, primarily as a growth inducing, tissue regenerating or wound healing agent.

Accordingly, the invention provides:
i) The use of a binding protein of the invention together with IGF in free or fixed combination for stimulating the growth of a subject, tissue or organ regeneration or wound healing, or
ii) a method of stimulating the growth of a subject, tissue or organ regeneration or wound healing in a subject which comprises administering a therapeutically effective amount of a binding protein of the invention together with a therapeutically effective amount of an IGF to a patient in need of such treatment, or
iii) a pharmaceutical composition for stimulating the growth of a subject, tissue or organ regeneration or wound healing which comprises a binding protein of the invention together with an IGF and with a pharmaceutically acceptable carrier or diluent, or
iv) a twin-pack containing separate unit dose forms of a binding protein of the invention and an IGF, together with instructions for mixing or concomitant administration.

In association with an IGF, a binding protein of the invention is of special interest for mediating chondrogenesis or hematopoieses. This may be shown in the following tests A to C:
A) An IGF increases bone formation as indicated by e.g. an increased incorporation of [3H]proline into collagen and non-collagen proteins in fetal rat calvaria. Surprisingly, a synergistic effect occurs when an IGF is used in the presence of a binding protein of the invention. Organ cultures of rat calvaria are prepared by dissecting frontal and parietal bones from 21-day old fetal rats, splitting along the sagittal suture and culturing according to the method of Kream et al. (Endocrinology (1985) 116, 296). A binding protein or IGF is added in doses from 10 to 200 ng/ml of cultures. When they are added in combination to each other the molar ratio is 1:1. Culturing is effected for 24 to 48 hours. To quantitate the incorporation of [3H]proline into collagenase-digestable protein and non-collagen protein, bone homogenates are digested with bacterial collagenase according to the method of Diegelman R. and Peterkofsky (Dev. Biol. (1972) 28:443) and modified by Kream et al. (Endocrinology (1985) 116:296).
B) An IGF decreases bone resorption as indicated by a decrease in release of [45]Ca from bone. Surprisingly, a synergistic effect occurs when an IGF is used in the presence of a binding protein of the invention. The test is effected according to the principles of Raisz (J. Clin. Invest. (1965) 44, 103). Pregnant rats are injected s.c. with [45]Ca on the eighteenth day of gestation. An IGF, alone or in the presence of a binding protein of the invention, is injected at a dose of 10 ng to 200 ng per animal. The binding protein is added so that the molar ratio of IGF is 1:1. On day nineteen, the animals are sacrified, the fetuses removed. The mineralized shafts of the radii and ulnae are dissected and placed in culture. Resorption is quantitated on the basis of release of [45]Ca from the bone explants.
C) It has also been found that the IGF-binding proteins of the invention as well as other IGF-binding proteins potentiate the erythropoetin-like effect of IGF-I. This may be, in particular, demonstrated by testing IGF-I, e.g. 10 ng/ml IGF-I, alone and in combination with the mature IGF binding protein of Figure 2, e.g. a 50 »l aliquot of a supernatant derived from a culture of a CHO cell line expressing the mature IGF binding protein of Figure 2, in a CFU-E assay as described in Fagg, B. Roitsch, C.A., Cell. Physiol. (1986) 126 : 1.Whereas the result obtained with IGF-binding protein alone is not significantly different from the control, a synergistic effect of the combination is seen when compared to IGF-I alone.

Further, the mitogenic activity of an IGF combined with a binding protein of the invention may be tested as follows: The incorporation of [³H] methyl-thymidine into CCL 39 cells (Chinese hamster lung fibroblasts) in culture is measured as described by Plouet et al, Cell. Mol. Biol. (1984) 30: 105. In this assay, cell line CCl 39 is seeded in a plate at 40 000 cells per well in 0.5 ml MEM culture medium (Gibco) containing 10% fetal calf serum, 0.1% penicillin, 0.4% streptomycin and 0.5% fungizone. After 72 hours incubation at 37°C in an atmosphere loaded with 5% CO₂, cells are washed with MEM medium in the absence of fetal calf serum and then cultured in this medium for 20 hours. At this stage, the cell culture is confluent and an IGF or a binding protein or both together are inoculated each at a dose of 10 ng to 200 ng culture medium. When added together the molar ratio must be 1:1. The test sample is incubated at 37°C for 24 hours and then added with 1»Ci [³H] methylthymidine in 10»l PBS. After 4 hours incubation the incorporation of methylthymidine is stopped by washing cells with PBS. Cells are fixed with 0.5 ml trichloroacetic acid (5%) for 30 min, washed with water and finally lysed with 0.5 ml of NaOH 0.1M for 2 hours at 37°C. 0.5 ml of lysate is transferred into a scintillation flask and mixed with 3 ml of scintillation liquid for measuring β-radioactivity. The binding protein potentiates the mitogenic activity of IGF although the radioactivity level that is measured when a binding protein is used alone is not substantially different from that of the control sample.

More particularly a binding protein of the invention, in combination with an IGF, is useful a) for treating hypopituitarism, Laron-type dwarfism, osteoporosis, anemias especially complications following an chronic renal failure and liver or kidney deficiency and b) for promoting healing of wounds such as ulcers and burns or those occuring in accidental events or resulting from surgery.

For use in association with a binding protein of the invention, IGF is preferably selected from IGF-I as described in Rinderknecht, E. and Humbel, R.E., J. Biol. Chem. (1978) 253: 2769, IGF-II as described in Rinderknecht, E. and Humbel, R.E., FEBS (1978) 89: 283 and any derivative or fragment of IGF-I and IGF-II having an insulin-like growth factor activity. Most preferably, this is IGF-II.

For use in association with an IGF, a binding protein of the invention is preferably a protein which is from 85% to 100% homologous with pre IGF-BP or IGF-BP as shown in Figure 2.

When not associated with IGFs, binding proteins of the invention have further therapeutic applications in any physiological disorders resulting from an excessive production of free IGFs, e.g. IGF-producing cancers such as breast or kidney cancer, diabetic proliferative retinopathy or abnormal growth of tall children with high serum level of free IGF.

Accordingly, the invention also provides:
i) the use of a binding protein of the invention for treating physiological disorders resulting from an excessive production of free IGF by a mammalian, for example human body, e.g. IGF-producing cancers, diabetic retinopathy or abnormal growth of tall subjects, or
ii) a method of treating physiological disorders resulting from an excessive production of free IGF, e.g. IGF-producing cancers, diabetic retinopathy or abnormal growth of a subject which comprises administering a therapeutically effective amount of a binding protein of the invention to a subject in need of such treatment, or
iii) a pharmaceutical composition for treating physiological disorders resulting from an excessive production of free IGF, e.g. IGF-producing cancers, diabetic retinopathy or abnormal growth of a subject which comprises a binding protein of the invention in association with a pharmaceutically acceptable carrier or diluent.

Fragments of mutated forms of the pre-IGF-BP or IGF-BP as shown in Figure 2 are of particular value for treating the physiological disorders resulting from an excessive production of free IGF in the human body.

A binding protein of the invention, alone or in combination with an IGF, may be administered by any conventional route suitable for peptides, or particular enterally, e.g. in the form of tablets or capsules or, preferably parenterally, e.g. subcutaneously or intravenously in the form of injections of infusions. Further, it may be also used topically, e.g. in the form of ointments or suspensions when used, e.g. as a wound healing agent.

For all the above indications the appropriate dosage will be course vary depending upon, for example, the nature and severity of the disorder to be treated and the mode of administration. For example, satisfactory results may be obtained in the treatment of osteoporosis or anemia at daily dosages from about to 0.1 »g/kg to 40 »g/kg body weight, preferably from about 0.5 »g/kg to about 20 »g/kg body weight of a binding protein of the invention. In larger mammals, for example humans, an indicated daily dosage is from about 5 »g to about 500 »g, preferably from 10 to 100 »g conveniently adminstered parenterally, for example once a day. For wound healing, a daily dose of from 0.1 to 10 »g of a protein of the invention per cm2 wound area is suitably indicated in larger mammals, for example humans. This is conveniently administered once a day. When used in combination with an IGF, the molar ratio of the binding protein to IGF is preferably from 0.1:1 to 5:1, more preferably from 0.5:1 to 2:1, most preferably 1:1.

Pharmaceutical compositions of the invention may be manufactured in conventional manner.

## Claims (Claims for the following Contracting State(s): AT, BE, CH, DE, FR, GB, IT, LI, LU, NL, SE)

1. An Insulin-like Growth Factor-binding protein comprising an amino acid sequence which is 95% to 100% homologous to the amino acid sequence of Figure 2 starting at position 1 and ending at position 289.

2. An IGF binding protein according to claim 1 comprising the amino acid sequence of Figure 2 starting either at position -39, -38 or 1 and ending at position 289.

3. A recombinant DNA fragment comprising a nucleotide sequence encoding an IGF-binding protein according to claim 1 or 2.

4. A DNA fragment according to claim 3 comprising the DNA sequence of Figure 2 starting at position 120, 123 or 237 and ending at position 1103.

5. A vector comprising a DNA fragment according to claim 3 or 4, capable of expressing the IGF-binding protein encoded by said DNA fragment in a procaryotic or eucaryotic host.

6. A procaryotic or eucaryotic cell line transformed either by the DNA fragment of claim 3 or 4 or by the vector according to claim 5.

7. A method for the production of an IGF-binding protein comprising culturing a cell line according to claim 6 and recovering the IGF-binding protein from the culture.

8. A pharmaceutical composition for stimulating the growth of a subject, tissue or organ regeneration or wound healing which comprises a binding protein according to claim 1 or 2 together with an IGF and with a pharmaceutically acceptable carrier or diluent.

9. A pharmaceutical composition for treating physiological disorders resulting from an excessive production of free IGF which comprises a binding protein according to claim 1 or 2 in association with a pharmaceutically acceptable carrier or diluent.

## Claims (Claims for the following Contracting State(s): ES, GR)

1. A process for the production of an Insulin-like Growth Factor (IGF)-binding protein comprising culturing a cell line, transformed with a DNA fragment which codes for an IGF-binding protein comprising an amino acid sequence which is 95% to 100% homologous to the amino acid sequence of Figure 2 starting at position 1 and ending at position 289, to express the IGF-binding protein and recovering the IGF-binding protein from the culture.

2. A process for the preparation of a pharmaceutical composition for stimulating the growth of a subject, tissue or organ regeneration or wound healing comprising admixing an IGF-binding protein, having an amino acid sequence which is 95% to 100% homologous to the amino acid sequence of Figure 2 starting at position 1 and ending at position 289, together with an IGF and a pharmaceutically acceptable carrier or diluent.

3. A process for the preparation of a pharmaceutical composition for treating physiological disorders resulting from excessive production of free IGF comprising admixing an IGF-binding protein, having an amino acid sequence which is 95% to 100% homologous to the amino acid sequence of Figure 2 starting at position 1 and ending at position 289, with a pharmaceutically acceptable carrier or diluent.

## Patentansprüche (Patentansprüche für folgende(n) Vertragsstaat(en): AT, BE, CH, DE, FR, GB, IT, LI, LU, NL, SE)

1. Bindeprotein für insulinähnlichen Wachstumsfaktor, das eine Aminosäuresequenz umfaßt, die zu 95 % bis 100 % zur Aminosäuresequenz von Figur 2 homolog ist, die an Position 1 beginnt und an Position 289 endet.

2. IGF-Bindeprotein nach Anspruch 1, das die Aminosäuresequenz von Figur 2 umfaßt, die entweder an Position -39, -38 oder 1 beginnt und an Position 289 endet.

3. Rekombinantes DNA Fragment, das eine Nukleotidsequenz umfaßt, die ein IGF-Bindeprotein nach Anspruch 1 oder 2 kodiert.

4. DNA Fragment nach Anspruch 3, das die DNA Sequenz von Figur 2 umfaßt, die an Position 120, 123 oder 237 beginnt und an Position 1103 endet.

5. Ein DNA Fragment nach Anspruch 3 oder 4 enthaltender Vektor, der zur Expression des IGF-bindenden Proteins in einem prokaryontischen oder eukaryontischen Wirt fähig ist, das durch dieses DNA Fragment kodiert wird.

6. Prokaryontische oder eukaryontische Zellinie, die entweder durch das DNA Fragment nach Anspruch 3 oder 4 oder durch den Vektor nach Anspruch 5 transformiert ist.

7. Verfahren zur Herstellung eines IGF-bindenden Proteins, gekennzeichnet durch Kultivierung einer Zellinie nach Anspruch 6 und Gewinnung des IGF-Bindeproteins aus der Kultur.

8. Pharmazeutische Zusammensetzung zur Wachstumsstimulation eines Patienten, Gewebs- oder Organregeneration oder Wundheilung, die ein Bindeprotein nach Anspruch 1 oder 2 zusammen mit einem IGF und mit einem pharmazeutisch annehmbaren Träger oder Verdünnungsmittel enthält.

9. Pharmazeutische Zusammensetzung zur Behandlung von physiologischen Störungen, die aus übermäßiger Bildung von freiem IGF resultieren, die ein Bindeprotein nach Anspruch 1 oder 2 zusammen mit einem pharmazeutisch annehmbaren Träger oder Verdünnungsmittel enthält.

## Patentansprüche (Patentansprüche für folgende(n) Vertragsstaat(en): ES, GR)

1. Verfahren zur Herstellung eines Bindeproteins eines insulinähnlichen Wachstumsfaktors (IGF), gekennzeichnet durch Kultivierung einer Zellinie, die mit einem DNA Fragment transformiert ist, das für ein IGF-Bindeprotein kodiert, welches eine Aminosäuresequenz umfaßt, die zu 95 % bis 100 % homolog zu der Aminosäuresequenz der Figur 2 ist, welche an Position 1 beginnt und an Position 289 endet, um das IGF-bindende Protein zu exprimieren, und durch Gewinnung des IGF-bindenden Proteins aus der Kultur.

2. Verfahren zur Herstellung einer pharmazeutischen Zusammensetzung zur Wachstumsstimulation eines Patienten, Gewebs- oder Organregeneration oder Wundheilung, gekennzeichnet durch Mischen eines IGF-bindenden Proteins, das eine Aminosäuresequenz aufweist, die zu 95 % bis 100 % homolog zur Aminosäuresequenz von Figur 2 ist, welche an Position 1 beginnt und an Position 289 endet, zusammen mit einem IGF und einem pharmazeutisch annehmbaren Träger oder Verdünnungsmittel.

3. Verfahren zur Herstellung einer pharmazeutischen Zusammensetzung zur Behandlung von physiologischen Störungen, die von einer übermäßigen Bildung von freiem IGF herrühren, gekennzeichnet durch Mischen eines IGF-bindenden Proteins, das eine zu 95 % bis 100 % zur Aminosäuresequenz der Figur 2 homologe Aminosäuresequenz aufweist, die an Position 1 beginnt und an Position 289 endet, mit einem pharmazeutisch annehmbaren Träger oder Verdünnungsmittel.

## Revendications (Revendications pour l'(les) Etat(s) contractant(s) suivant(s): AT, BE, CH, DE, FR, GB, IT, LI, LU, NL, SE)

1. Protéine de liaison de facteurs de croissance analogues à l'insuline (IGF), comprenant une séquence d'acides aminés homologue de 95% à 100% à la séquence d'acides aminés de la figure 2, commençant à la position 1 et finissant à la position 289.

2. Protéine de liaison de IGF selon la revendication 1, comprenant la séquence d'acides aminés de la figure 2, commençant à la position -39, -38 ou 1 et finissant à la position 289.

3. Fragment d'ADN recombinant comprenant une séquence nucléotidique qui code pour une protéine de liaison de IGF selon la revendication 1 ou 2.

4. Fragment d'ADN selon la revendication 3, comprenant la séquence d'ADN de la figure 2, commençant à la position 120, 123 ou 237, et finissant à la position 1103.

5. Vecteur comprenant un fragment d'ADN selon la revendication 3 ou 4 capable d'exprimer la protéine de liaison de IGF codée par ledit fragment d'ADN, chez un organisme hôte procaryote ou eucaryote.

6. Lignée cellulaire procaryote ou eucaryote transformée à l'aide du fragment d'ADN selon la revendication 3 ou 4, ou du vecteur selon la revendication 5.

7. Procédé de production d'une protéine de liaison de IGF comprenant la mise en culture d'une lignée cellulaire selon la revendication 6 et la récupération de la protéine de liaison de IGF à partir de la culture.

8. Composition pharmaceutique destinée à stimuler la croissance d'un sujet, la régénération d'un tissu ou d'un organe, ou la cicatrisation , comprenant une protéine de liaison selon la revendication 1 ou 2, ainsi qu'un IGF et un véhicule ou diluant pharmaceutique acceptable.

9. Composition pharmaceutique destinée à traiter les troubles physiologiques résultant d'une production excessive de IGF libres, comprenant une protéine de liaison selon la revendication 1 ou 2, en association avec un véhicule ou diluant pharmaceutiquement acceptable.

## Revendications (Revendications pour l'(les) Etat(s) contractant(s) suivant(s): ES, GR)

1. Procédé de production d'une protéine de liaison de facteurs de croissance analogues à l'insuline (IGF), comprenant la mise en culture d'une lignée cellulaire transformée avec un fragment d'ADN qui code pour une protéine de liaison de IGF comprenant une séquence d'acides aminés homologue de 95% à 100% à la séquence d'acides aminés de la figure 2, commençant à la position 1 et finissant à la position 289, pour exprimer la protéine de liaison de IGF, et la récupération de la protéine de liaison de IGF à partir de la culture.

2. Procédé de préparation d'une composition pharmaceutique destinée à stimuler la croissance d'un sujet, la régénération d'un tissu ou d'un organe ou la cicatrisation, qui consiste à mélanger une protéine de liaison de IGF ayant une séquence d'acides aminés homologue de 95% à 100% à la séquence d'acides aminés de la figure 2 commençant à la position 1 et finissant à la position 289, avec un IGF et un véhicule ou diluant pharmaceutiquement acceptable.

3. Procédé de préparation d'une composition pharmaceutique destinée à traiter les troubles physiologiques résultant d'une production excessive de IGF libres, qui consiste à mélanger une protéine de liaison de IGF ayant une séquence d'acides aminés homologue de 95% à 100% à la séquence d'acides aminés de la figure 2, commençant à la position 1 et finissant à la position 289, avec un véhicule ou diluant pharmaceutiquement acceptable.
